# EUROPÄISCHE PATENTANMELDUNG

(11) **EP 0 638 329 A1**
(43) Veröffentlichungstag der Anmeldung: **15.02.1995**
(21) Anmeldenummer: 93112615.5
(22) Anmeldetag: 06.08.1993
(51) Int. Cl.: A61N 1/05

(54) **Therapiegerät zur Behandlung von Harninkontinenz**

(71) Anmelder: Barsom, Shafik, Dr. med., D-30177 Hannover (DE); Kluge, Paul, D-30519 Hannover (DE)
(72) Erfinder: Barsom, Shafik, Dr. med., D-30177 Hannover (DE); Kluge, Paul, D-30519 Hannover (DE)
(74) Vertreter: Arendt, Helmut, Dipl.-Ing.

(57) **Zusammenfassung**

Zur Behandlung von Harninkontinenz mit einer in einem Gehäuse angeordneten Spannungsquelle und einer Impulsgeberschaltung zur Erzeugung elektrischer Spannungsimpulse wird ein medizines Therapiegerät geschaffen, das eine einfache und über längere Zeiträume andauernde sichere Anwendung zur Stimulierung größerer Muskel- und Nervenbereiche ermöglicht. Zu diesem Zweck ist vorgesehen, das Gehäuse des Gerätes aus mehreren Teilen zusammenzusetzen, von welchen wenigstens eines einen Aufnahmeraum für die Spannungsquelle aufweist. In einem weiteren Aufnahmeraum ist die Impulsgeberschaltung und ein in der Verbindungsleitung zwischen der Spannungsquelle und der Impulsgeberschaltung vorgesehener, in Ruhestellung geschlossener Reedkontakt als Betriebsschalter angeordnet. Ferner ist das Gerät mit drei, die Spannungsimpulse abgebenden Elektroden ausgerüstet, von welchen eine als Pluspol und zwei untereinander verbundene Elektroden als Minuspol ausgebildet sind. In einem für das Gerät vorgesehenen Aufbewahrungsbehälter bei Nichtgebrauch ist ein Magnet zum öffnen des Reedkontaktes angeordnet.

## Beschreibung

Die Erfindung betrifft ein elektrisches Therapiegerät zur Behandlung von Harninkontinenz, bestehend aus einem in den Körper des Patienten einführbaren Gehäuse, in dem eine von einer in dem Gehäuse angeordneten Spannungsquelle gespeiste Schaltungsanordnung (Impulsgeberschaltung) zur Erzeugung elektrischer Spannungsimpulse vorgesehen ist, welche über ringförmige, am Gehäuseaußenmantel angebrachte Elektroden abgegeben werden, und mit einem Betriebsschalter zum Ein- und Ausschalten des Gerätes.

Ein Gerät der vorgenannten Gattung ist durch die DE-OS 38 27 232 bekannt. Es ist ein Stimulator mit einem im Gehäuse angeordneten, von außen zu bedienenden Betriebsschalter zum Ein- und Ausschalten des Gerätes. Da das Gerät nach jeder Anwendung zu sterilisieren ist, besteht die Gefahr, daß ein solcher Schalter beschädigt und funktionsunfähig wird. Die Massekontaktgabe erfolgt störanfällig über einen am Gehäuseende befindlichen Schraubdeckel. Insgesamt ist die Gefahr von Betriebsstörungen bei der bekannten Ausführung erheblich. Außerdem ist das Gerät nicht in der Lage, einen größeren Muskel- und Nervenbereich zu stimulieren.

Der Erfindung liegt die Aufgabe zugrunde, ein Gerät der eingangs genannten Gattung für die Behandlung von Harninkontinenz zu schaffen, das eine einfache, auch über längere Zeiträume andauernde sichere Anwendung zur Stimulierung größerer Muskel- und Nervenbereiche ermöglicht, ohne daß der Patient besondere Bedienungsmaßnahmen zu beachten hat. Die Erfindung als Lösung zeichnet sich dadurch aus, daß das Gehäuse des Gerätes aus mehreren Teilen zusammengesetzt ist, von welchen wenigstens eines einen Aufnahmeraum für die Spannungsquelle aufweist, daß in einem weiteren Aufnahmeraum eines der Gehäuseteile die Impulsgeberschaltung und ein in der Verbindungsleitung zwischen der Spannungsquelle und der Impulsgeberschaltung vorgesehener, in Ruhestellung geschlossener Reedkontakt als Betriebsschalter angeordnet ist, daß das Gerät mit drei die Spannungsimpulse abgebenden Elektroden ausgerüstet ist, von welchen eine als Plus-Pol und zwei untereinander verbundene Elektroden als Minus-Pol ausgebildet sind, und daß für das Gerät zur Verwahrung bei Nichtgebrauch ein Aufbewahrungsbehälter vorgesehen ist, der einen Magneten zum öffnen des Reedkontaktes aufweist.

Der Einsatz eines Reedkontaktes innerhalb des Gehäuses, der in Ruhestellung geschlossen ist und bei Einwirkung eines Magnetfeldes geöffnet wird, hat den Vorteil, daß die Ein- und Ausschalteinrichtung durch einen Sterilisierungsvorgang nicht beschädigt und damit außer Funktion gesetzt werden kann. Als Spannungsquelle kann ein Akkumulator dienen. Der Aufbewahrungsbehälter ist mit einer Ladesteuerschaltung zur Steuerung des Ladeprozesses und zusätzlich mit einer Anschlußbuchse für ein Steckernetzgerät ausgerüstet. Der Ladevorgang wird durch den Magneten, beispielsweise einen Pergamentmagneten, im Aufbewahrungsbehälter gestartet, der mit Hilfe des geräteinternen Reedkontaktes die Impulsgeberschaltung im Gerät ausschaltet und die Ladeelektroden mit dem Akku verbindet. Die im Aufbewahrungsbehälter eingebaute Ladesteuerschaltung steuert den Ladeprozeß und verhindert ein Überladen des Akkumulators, falls das Gerät zu lange im Aufbewahrungsbehälter gelassen wird.

Für die Anwendung des Gerätes ist kein Zubehör erforderlich. Ebenso ist die Anwendung beim angeschlossenen Steckernetzgerät ausgeschlossen. Die Akkumulatoren sind hermetisch verschlossen. Bei einer korrekten Ladung besteht nicht die Gefahr des Austritts flüssigen Elektrolyts oder anderer giftiger Bestandteile. Die einzelnen Akkuzellen sind in einem Block durch einen die Akkus eng umschließenden Schrumpfschlauch zusammengefaßt.
Während der Anwendung hat das Gerät keinen Kontakt zu externen zusätzlichen Geräten, sondern ist auf Grund der internen Spannungsquelle autark. Die aus dem Gehäuse herausragenden Ladeelektroden sind während der Behandlung zum Schutz der Patienten ausgeschaltet. Da es praktisch keine Serviceteile, d. h. keine für die Wartung durch den Patienten betätigbare Stell- und Bedienungselemente im Innenraum des Gerätes gibt und dieses allein durch die Entnahme aus dem Aufbewahrungsbehälter betriebsbereit ist, kann von einem automatischen Anwendungsvorgang gesprochen werden.

Weitere, den Erfindungsgegenstand vorteilhaft gestaltende Merkmale sind in den Ansprüchen enthalten.

In der Zeichnung ist ein Ausführungsbeispiel der Erfindung schematisch dargestellt und nachstehend erläutert. Es zeigen:

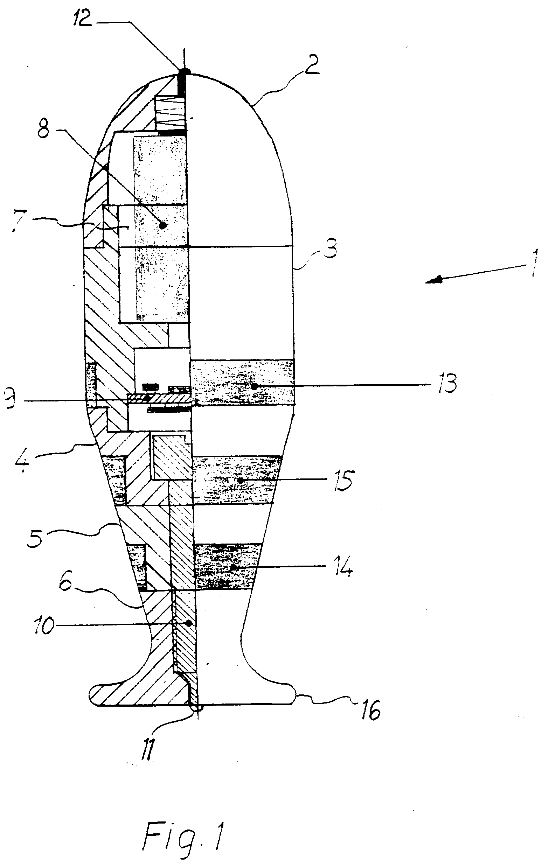

- Figur 1: die Seitenansicht eines Gerätes, zum Teil im Längsschnitt,
- Figur 2: ein Schaltbild der Impulsgeberschaltung und
- Figur 3: ein Schaltbild der Ladeschaltung.

Das Therapiegerät 1 besteht aus einem mehrteiligen Gehäuse mit den Gehäuseteilen 2, 3, 4, 5 und 6. Die Gehäuseteile 2, 3 und 4 sind zur hermetischen Abdichtung miteinander verklebt. In dem durch die Gehäuseteile 2 und 3 gebildeten Aufnahmeraum 7 ist beispielsweise ein NC-Akkumulator 8 gelagert. Er ist mit der auf der Platine 9 angeordneten Impulsgeberschaltung über einen nicht dargestellten Reedkontaktschalter elektrisch verbunden.

Die Gehäuseteile 4, 5, 6, sie bilden den hinteren Bereich des Gehäuses, sind durch einen als Zuganker wirkenden Schraubbolzen ebenfalls hermetisch dichtend gegeneinandergezogen. Das hintere Ende des Schraubbolzen 10 reicht aus dem abschließenden Gehäuseteil 6 heraus und dient als positive Ladeelektrode 11. Aus Gründen der Übersichtlichkeit ist auf die Darstellung der Verbindung zwischen dem Akkumulator 8 und dem Schraubbolzen 10 verzichtet worden. Am vorderen Ende des Gehäuses ist die negative Ladeelektrode 12 des Akkumulators angeordnet.

In den Außenmantel des Gerätegehäuses sind drei Ringelektroden 13, 14 und 15 eingelassen. Von diesen sind die Elektroden 13 und 14 als negative Elektroden miteinander verbunden, während sich zwischen Ihnen die positive Elektrode 15 befindet. Über diese Elektroden werden die Spannungsimpulse zur Stimulierung der Muskel- und Nervenzellen abgegeben.

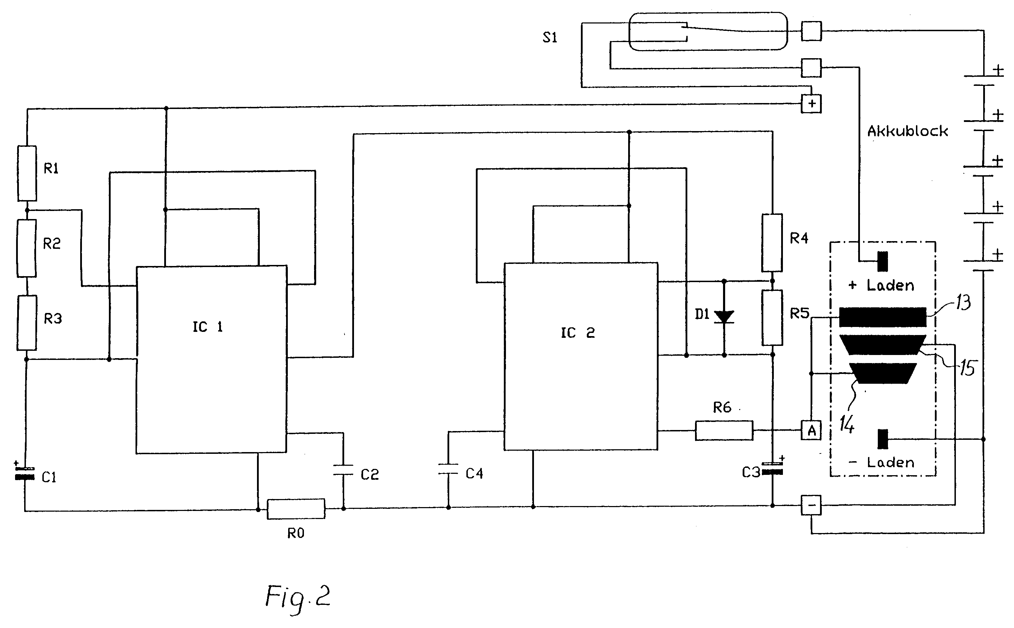

Um eine körpergerechte Anpassung zu erreichen, ist das Gehäuse um seine Längsachse rotationssymmetrisch ausgebildet. Es zeigt im vorderen Bereich eine Abrundung und im hinteren Bereich eine Einschnürung mit einer progressiven Durchmesserzunahme am Ende, das gleichzeitig als Griffstück dient. Die Impulsgeberschaltung (Figur 2) wird für den Betrieb durch den Reedkontakt S1 mit einem Akkublock aus 5 Zellen mit jeweils 1,2 V Nennspannung und einer typischen Kapazität von 75 mAh (Nennkapazität 60 mAh) verbunden. Das IC 1 arbeitet als astabiler Multivibrator mit einer Einschaltdauer von 90 s und einer Pause von 60 s. Die die Zeitkonstanten bestimmenden Bauteile sind die Widerstände R 1, R 2 und R 3 sowie C 1. Der kapazitive Widerstand C 2 dient der Glättung der schaltkreisinternen Schwellspannung und trägt somit zur Stabilität der Zeitkonstanten bei. Während des Einschaltzyklus arbeitet das IC 2 ebenfalls als astabiler Multivibrator mit 0,33 s Impulseinschaltdauer und 3,3 s Pause. Die die Zeitkonstanten bestimmenden Bauteile sind die Widerstände R 4, R 5 und C 3. Der Ausgang des IC 2 ist über R 6 mit den Patientenelektroden verbunden. In deren Mitte befindet sich die Masseelektrode 15. Bei einem minimal anzunehmenden Patientenersatzinnenwiderstand von 1 K Ω begrenzt R 6 den Stimulationsstrom auf 2 mA. Im Fehlerfall (Kurzschluß des Ausgangs mit der Versorgungsspannung) fließt ein maximaler Patientenstrom von 6 mA. Die Diode D 1 verbessert das Ladeverhalten von C 3 und die Impulsform am Ausgang des C 2. Der Widerstand R0 ist aus Layoutgründen in die Schaltung eingefügt und hat keine elektrische Funktion. Der Kondensator C 4 hat dieselbe stabilisierende Wirkung für den Multivibrator IC 2 wie der bereits für den IC 1 beschriebene Kondensator C 2.

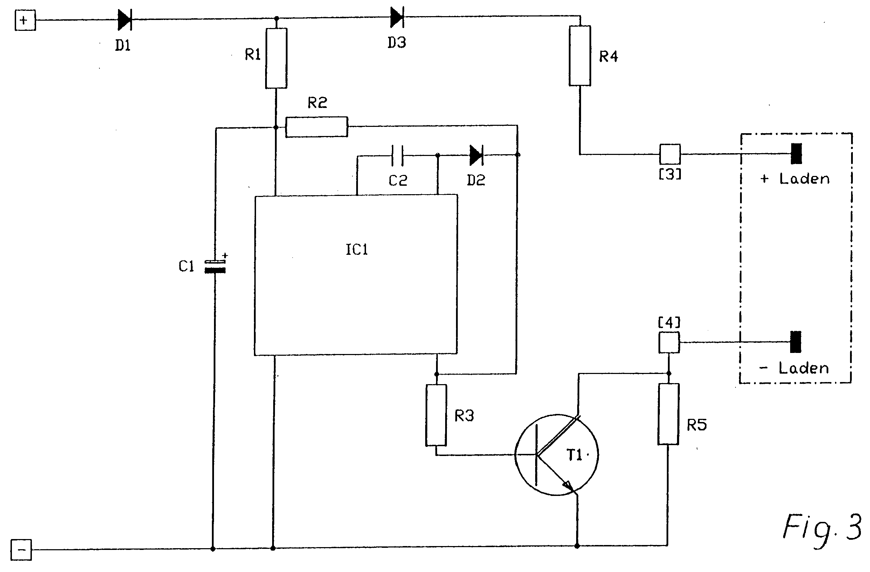

Die Ladeschaltung (Figur 3) wird durch ein Steckernetzteil mit einem Klinkenstecker über die Klinkenbuchse am Aufbewahrungsbehälter mit Betriebsspannung versorgt. Die Diode D 1 dient als Verpolungsschutz. Die Widerstände R 1 und C 2 versorgen den Ladesteuerbaustein mit einer von der Ladespannung entkoppelten und geglätteten Versorgungsspannung. Die Diode D 3 dient dem Entlade- und Verpolungsschutz für die Akkumulatoren im Gerät. Der Widerstand R 4 grenzt den Ladestrom auf 5 mA, die Widerstände R 4 und R 5 den Ladeerhaltungsstrom auf 0,5 mA. Die Widerstände R 2, C 2 bestimmen die Zeitkonstante für die Ladedauer.

Der Akkumulator wird nach dem Einstecken des Gerätes bei einer Ladedauer von 14 h mit 5 mA geladen und nach dem Ausschalten des Ladestroms durch den Steuerbaustein über die Widerstände R 4 und R 5 mit einem Ladeerhaltungsstrom versorgt. Der Widerstand R 3 und der Thyristor T 1 steuern den Ladestrom. Die Diode D 2 legt die Schaltwechsel für den schaltkreisinternen Timer fest.

## Patentansprüche

1. Elektrisches Therapiegerät zur Behandlung von Harninkontinenz, bestehend aus einem in den Körper des Patienten einführbaren Gehäuse, in dem eine von einer in dem Gehäuse angeordneten Spannungsquelle gespeiste Schaltungsanordnung (Impulsgeberschaltung) zur Erzeugung elektrischer Spannungsimpulse angebracht ist, welche über ringförmige, am Gehäuseaußenmantel vorgesehene Elektroden zur Muskel- und Nervenstimulanz nach außen abgegeben werden, und mit einem Betriebsschalter zum Ein- und Ausschalten des Gerätes, dadurch gekennzeichnet, daß das Gehäuse aus mehreren Teilen (2 - 6) zusammengefügt ist, von welchen wenigstens eines einen Aufnahmeraum (7) für die Spannungsquelle (8) aufweist,
daß in einem weiteren Aufnahmeraum (17) die Impulsgeberschaltung und ein in der Verbindungsleitung zwischen der Spannungsquelle und der Impulsgeberschaltung vorgesehener, in Ruhestellung geschlossener Reedkontakt als Betriebsschalter angeordnet sind,
daß das Gerät mit drei, die Spannungsimpulse abgebenden Elektroden (13, 14, 15) ausgerüstet ist, von welchen eine (15) als Plus-Pol und zwei untereinander verbundene Elektroden (13, 14) als Minus-Pole ausgebildet sind, und daß für das Gerät bei Nichtgebrauch ein Aufbewahrungsbehälter vorgesehen ist, der einen Magneten zum öffnen des Reedkontaktes aufweist.

2. Gerät nach Anspruch 1 dadurch gekennzeichnet, daß die Spannungsquelle ein aufladbarer Akkumulator (8) ist, der mit an den Gehäuseenden angeordneten, während der Anwendung zum Schutz des Patienten ausgeschalteten Ladekontakten (11, 12) verbunden ist.

3. Gerät nach Anspruch 2 dadurch gekennzeichnet, daß der Aufbewahrungsbehälter eine Ladesteuerschaltung zur Steuerung des Ladeprozesses und eine Anschlußbuchse für ein Steckernetzgerät aufweist.

4. Gerät nach einem der Ansprüche 1 bis 3 dadurch gekennzeichnet, daß die Gehäuseteile (2, 3, 4) miteinander verklebt sind.

5. Gerät nach einem der Ansprüche 1 bis 4 dadurch gekennzeichnet, daß wenigstens einige der Gehäuseteile (4, 5, 6) durch eine Bolzenschraube (10) hermetisch dichtend gegeneinandergezogen werden, deren eines Ende als Ladekontaktelektrode (11) nach außen reicht.

6. Gerät nach einem der Ansprüche 1 bis 5 dadurch gekennzeichnet, daß das Gehäuse um seine Längsachse rotationssymmetrisch ausgebildet ist und im Bereich des griffseitigen Endes (16) eine keilförmige Verjüngung mit einer anschließenden, überproportionalen Erweiterung aufweist.
